# EUROPEAN PATENT SPECIFICATION

(11) **EP 4 263 112 B1**
(45) Date of publication and mention of the grant of the patent: **22.01.2025**
(21) Application number: 21845096.3
(22) Date of filing: 21.12.2021
(51) Int. Cl.: B23K 26/03, A61F 9/04, B23K 26/21, B23K 26/32, B23K 26/70, G02B 21/00, G06F 3/01, G02B 27/01

(54) **WELDING WORKSTATION, VISION KIT FOR A WELDING WORKSTATION AND VISION METHOD FOR WELDING OPERATIONS**
SCHWEISSARBEITSPLATZ, SICHTKIT FÜR EINEN SCHWEISSARBEITSPLATZ UND SICHTVERFAHREN FÜR SCHWEISSARBEITEN
POSTE DE TRAVAIL DE SOUDAGE, KIT DE VISION POUR POSTE DE TRAVAIL DE SOUDAGE ET PROCÉDÉ DE VISION POUR OPÉRATIONS DE SOUDAGE

(30) Priority: 21.12.2020 IT 202000031727
(43) Date of publication of application: 25.10.2023
(73) Proprietor: Liga, Pietro, 10060 Cantalupa (TO) (IT); Liga, Sergio, 10043 Orbassano (TO) (IT); Bergadano, Massimo, Shanghai, 200040 (CN)
(72) Inventor: Liga, Pietro, 10060 Cantalupa (TO) (IT); Liga, Sergio, 10043 Orbassano (TO) (IT); Bergadano, Massimo, Shanghai, 200040 (CN)
(74) Representative: Studio Torta S.p.A.
(86) International application number: PCT/IB2021/062103
(87) International publication number: WO 2022/137116

(56) References cited:
- CN-A- 110 434 510
- CN-U- 203 944 992
- US-A1- 2019 047 068

## Description

The present invention relates to a welding and microwelding workstation, to a method of vision of welding operations and to a method of updating a welding workstation, see claims 1, 12 and 10 respectively

### STATE OF THE PRIOR ART

Welding workstations for manually carrying out precision welding or microwelding operations are known, for example, in the orthodontic and in the jewelery fields.

Such welding workstations (see for example CN 203 944 992 U) comprise, in general:
- a welding device, for example a laser welding device;
- a process chamber, within which the welding process is carried out by means of the welding device; and
- a vision system, which enables the operator to observe the inside of the process chamber while carrying out the welding.

In particular, the welding operation is carried out by an operator, who inserts his/her hands inside the process chamber and handles the objects to be welded and/or the welding device, observing the inside of the process chamber by means of the vision system.

The vision system can be, for example, a microscope comprising an objective arranged inside the process chamber and one or more eyepieces, which enable the operator to observe the objects arranged inside the process chamber on an enlarged scale. However, the use of the microscope obliges the operator to remain in a fixed position, with his/her eyes in contact with the eyepieces, for the whole duration of the welding operations. Such posture can be not very ergonomic, especially if kept for prolonged intervals of time, causing discomfort for the operator.

In fact, recent studies within the scope of the ergonomic risk assessment have highlighted the manifestation of muscle-skeletal disorders, eyestrain and mental fatigue due to postures similar to those assumed by the operators of the welding workstations.

Moreover, the use of the microscope as vision system can be even less ergonomic for the operators who wear reading glasses. In fact, such operators use the microscope after taking off their reading glasses, making use of the focusing settings of the instrument for adapting it to sight. However, the repeated taking off of the reading glasses from the face of the operator entails a further eyestrain due to the focusing at different distances.

The vision system can comprise, alternatively, a video camera with an objective arranged inside the process chamber and a screen, which is fixed outside the process chamber and projects the images filmed by the video camera. During the process, the operator carries out the welding operations keeping the visual contact with the screen. Therefore, also in this case, the operator is in a position which is not always ergonomic, since it is not possible for him/her to freely rotate his/her head without losing the visual contact with the screen.

It is further proper to note that the artificial or natural light present in the workplace can easily cause reflections on the screen, causing the risk of glaring the operator. Additionally, the quality of the images reproduced on the screen is not always optimal.

CN-A-110434510 discloses welding equipment based on a virtual reality technology and comprising a 3D scanner, a welding wire, a welding gun, a controllable mechanical arm, a universal ball, a control rod and a head-mounted stereoscopic display. CN-U-203944992U discloses an integrated jewelry laser spot welder. A space is formed below a mainframe, a cooling-water machine is located in the space, a gap is reserved between the cooling-water machine and the mainframe, and the cooling-water machine is in contact with the ground. US-A1-2019047068 discloses a welding apparatus including a multi-axis robotic arm having a first end; a welding tool attached to the first end; an image acquisition device attached to the first end and having a light filtering system; the image acquisition device is configured to monitor a welding target and to provide an image of the welding target to an operator; a control unit is configured to control the robotic arm and the welding tool; an input interface for a human operator is associated to the control unit and is configured to provide an input signal to the control unit and to control the robotic arm and welding tool substantially in real time.

Therefore, the need is felt to have a welding workstation where the manual welding operations can be carried out in an ergonomic manner even for prolonged periods of time.

The object of the present invention is to meet the above-described needs.

### SUMMARY OF THE INVENTION

The abovementioned object is achieved by a welding workstation, by a method of vision of manual welding operations on a welding workstation, and by a method of updating a welding workstation for carrying out manual welding operations as defined in claims 1, 12 and 10 respectively.

Further preferred embodiments of the present invention are defined in the appended claims.

### BRIEF DESCRIPTION OF THE DRAWINGS

To better understand the present invention, a preferred embodiment is described in the following, by way of non-limiting example and with reference to the accompanying drawings wherein:
- Figure 1 illustrates a perspective view of a welding workstation according to the present invention;
- Figure 2 illustrates a detail of the welding workstation of Figure 1 on an enlarged scale; and
- Figure 3 illustrates a side view of the welding workstation of Figure 1.

### DETAILED DESCRIPTION OF THE INVENTION

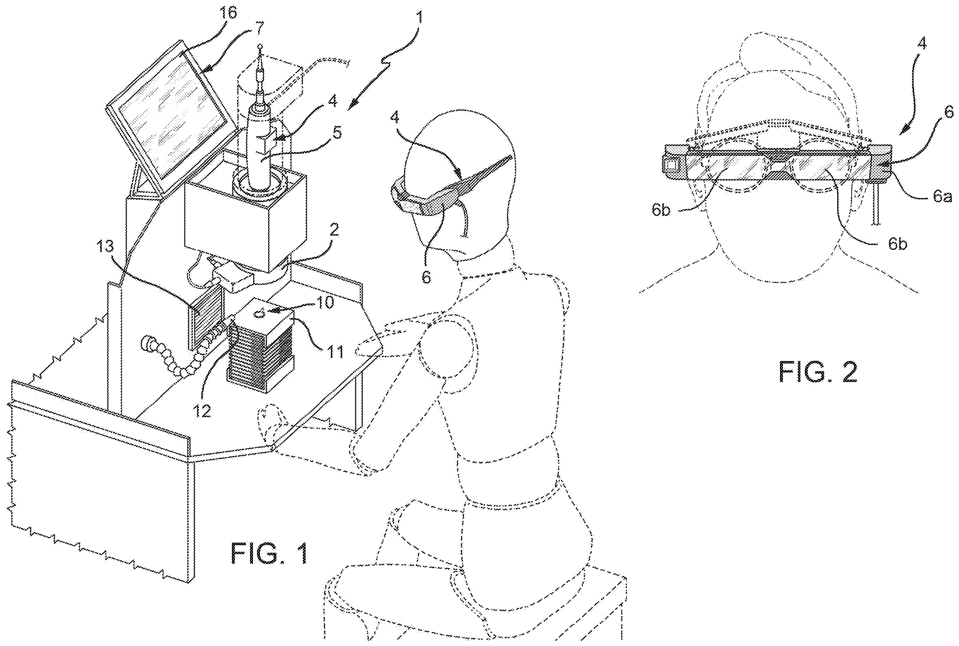

Figure 1 illustrates a welding workstation 1 for manually carrying out welding operations of objects 10 of various types.

In the following, welding process means any technology that allows jointing, under the action of heat and/or of pressure, two or more pieces made of the same material or of different materials. Specifically, the welding process can be carried out with or without weld material and with or without the melting of the objects 10 to be welded. On the other hand, the welding process can also be used for repairing a single object 10.

In particular, the objects 10 can be made, for example, of one or more metallic, ceramic or polymeric materials.

In the non-limiting embodiment shown, the welding workstation 1 is specifically used for the welding in the jewelery field. Consequently, the objects 10 can be made of precious or semi-precious materials and comprise precious stones or other minerals.

Alternatively, the welding workstation 1 is used in the prosthodontics/orthodontic fields, for example, for welding prostheses and hooks on skeletons; for jointing bridges, crowns, hooks or attachments; for correcting founding defects or for repairing prostheses.

The welding workstation 1 comprises a welding device 2 adapted to provide the necessary heat and/or pressure for jointing the objects 10 and a vision system 4 configured to show the objects 10 during the welding process to an operator.

According to the present invention, the welding device 2 comprises a laser source.

In particular, manual welding means a welding process that requires the manual intervention of an operator. However, such welding process could be partially automated. In the embodiment shown, such manual intervention consists in handling the objects 10 to be welded with respect to the laser source of the welding device 2. Alternatively or additionally, the manual intervention can consist, for example, in handling the welding device 2 or the weld material.

The welding workstation 1 further comprises a process chamber 3 within which the welding process of the objects 10 is carried out by means of the welding device 2.

The process chamber 3 comprises, in a known manner, a work platform 11, a nozzle 12 for inletting protective gases (argon, helium, carbon dioxide, nitrogen, etc.) and an extractor 13 for extracting the welding fumes.

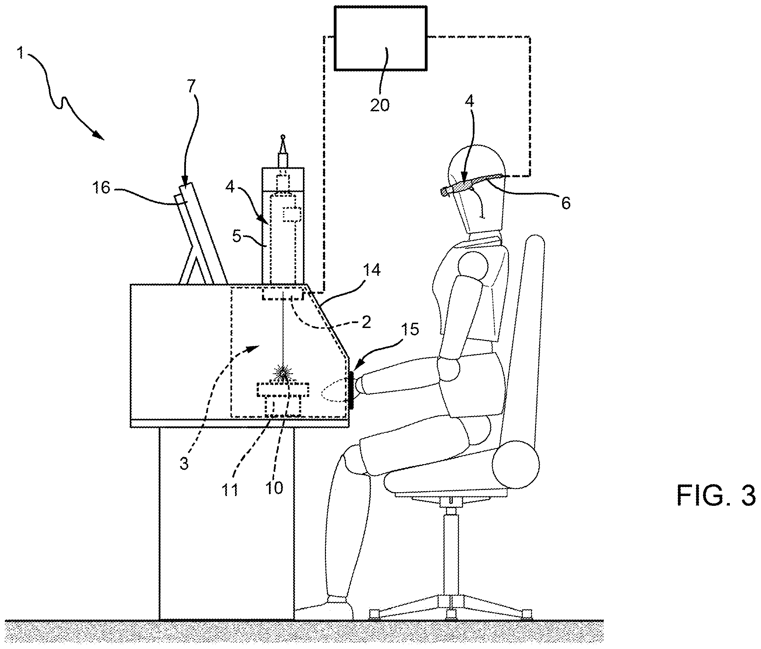

In the embodiment shown, the process chamber 3 is closed by a lid 14 and comprises, in a known manner, at least one opening 15 shaped for the insertion of the hands of the operator (Figure 3). Otherwise, the process chamber 3 could be open, i.e. not comprise the lid 14.

The vision system 4 comprises, in turn, an image acquisition device 5 and a viewer device 6 wearable by the operator and configured to show the images captured by the image acquisition device 5 to the operator.

In particular, the vision system 4 is configured to show the objects 10 arranged inside the process chamber 3 during the welding process to the operator.

The image acquisition device 5 and the wearable viewer device 6 further define a vision kit 100 (aspect not covered by the present invention) installable also on existing welding workstations.

Specifically, the image acquisition device 5 is a digital microscope or a video camera capable of capturing highly magnified images. In the case of existing welding workstations provided with a microscope, the video camera can be inserted in place of an eyepiece of the microscope.

Preferably, the image acquisition device 5 has a variable magnifying capacity superimposable to that of the optical microscopes.

The image acquisition device 5 is positioned at least partially inside the process chamber 3. The image acquisition device 5 has an objective - not shown - arranged inside the process chamber 3.

Alternatively, the image acquisition device 5 can be positioned outside the process chamber 3 so as to be able to film the objects 10 inside the chamber 3 (for example, through a transparent portion of the lid 14).

According to the present invention, the objective is further arranged coaxially to the laser source of the welding device 2 and, in particular, to the laser beam. This enables the operator to comfortably observe the points of the objects 10 to be invested with the laser radiation.

The image acquisition device 5 and the viewer device 6 are electrically connected to each other. In particular, they can be connected by means of a wired connection or, alternatively, by means of an electro-magnetic connection (for example, Bluetooth, infrared rays, radio waves, etc.).

In particular, the viewer device 6 comprises augmented reality glasses.

Such glasses comprise, in turn, a mount 6a and two lens elements 6b carried by the mount 6a. In particular, each lens element 6b is a screen, on which the images captured by the image acquisition device 5 are reproduced.

Similarly to the reading glasses, each lens element 6b is worn so as to be arranged in proximity of a respective eye of the operator.

Furthermore, the lens elements 6b can be arranged in a raised and peripheral position with respect to the field of view of the operator (as illustrated by the broken line in Figure 2). In this manner, the operator can stop looking at the images reproduced by the lens elements 6b without having to take off the viewer device 6.

Specifically, the lens elements 6b can be shifted with respect to the mount 6a up to being arranged in a raised position. More specifically, the lens elements 6b can be rotated with respect to the mount 6a around an axis parallel to the ground, i.e. horizontal with respect to the face of the operator.

The viewer device 6 further comprises a covering - not illustrated - removably couplable to the augmented reality glasses and, in particular, to the lens elements 6b on the opposite side of the eyes of the operator. Specifically, the lens elements 6b are transparent and the covering is opaque and its color is dark.

More specifically, the coupling of the covering to the lens elements 6b, improves the vision of the images reproduced by the lens elements 6b, since the covering acts as background for the images. Vice versa, when the covering is uncoupled from the lens elements 6b, the lens elements 6b - which are transparent - enable the operator to observe the space surrounding and outside the process chamber 3, without having to take off the augmented reality glasses.

By way of example, the covering is couplable to the augmented reality glasses by means of a magnetic connection.

Preferably, the two lens elements 6b enable the operator to obtain a stereoscopic vision of the images of the process chamber 3.

Furthermore, as illustrated in Figure 2, the viewer device 6 is wearable in addition to possible reading glasses worn by the operator.

The viewer device 6 can also be easily adjusted with the aim to correct any vision anomalies of the operator.

The welding workstation further comprises an electronic control unit 20 (schematically illustrated in Figure 3) electrically connected to the welding device 2 for controlling the process parameters thereof. The electronic control unit 20 is also electrically connected to the viewer device 6.

The electronic control unit 20 and the viewer device 6 are electrically connected by means of a wired connection or an electro-magnetic connection.

In particular, the viewer device 6 is configured to receive a signal associated with the values of the process parameters transmitted by the electronic control unit 20 and to enable the operator to view such parameters while carrying out the process. Specifically, each of the lens elements 6b can reproduce the values of such parameters in real time.

As the welding device 2 comprises, according to the present invention, a laser source, the process parameters controllable by means of the electronic control unit 20 can comprise one or more between, for example, the frequency and/or the power of the laser beam, the dimension of the focal spot, the welding speed, the focusing position.

The welding workstation 2 also comprises a process parameters adjustment device 7 electrically and operatively connected to the electronic control unit 20 for adjusting the process parameters.

In the embodiment shown, the process parameters adjustment device 7 is a screen 16 arranged outside the process chamber 3 and not wearable. Alternatively, the process parameters adjustment device 7 could be a joystick or a pedal operable by the operator.

According to a further embodiment, the process parameters adjustment device 7 could be integrated in the viewer device 6. In such case, the operator could adjust the process parameters by means of the process parameters adjustment device 7, for example through several push buttons positioned on the viewer device 6 or a vocal command.

Specifically, according to such further embodiment, the process parameters adjustment device 7 comprises a microphone - not illustrated - configured to receive a message uttered by the operator. Additionally, the process parameters adjustment device 7 is configured to transmit such message to the electronic control unit 20. The electronic control unit 20, in turn, is programmed to interpret the message uttered by the operator and consequently adjust the process parameters.

In particular, the microphone of the process parameters adjustment device 7 could be arranged at the viewer device 6.

Furthermore, the image acquisition device 5 is configured to simultaneously transmit the captured images to more than one viewer device 6 and/or to more external screens 16. Such further viewer devices 6 could be not placed in the workplace.

The operation of the welding workstation 1 is the following.

In use, the operator wears the viewer device 6 on his/her face, so that the two lens elements 6b are positioned at the eyes. Therefore, the operator can observe the images of the objects 10 to be welded captured by the image acquisition device 5 in a stereoscopic manner. In particular, the image acquisition device 5 captures the images of the inside of the process chamber 3.

Subsequently, the operator inserts his/her hands and the objects 10 to be welded in the process chamber 3 through the openings 15 (Figure 3).

At this point, the operator, viewing the objects 10 to be welded through the viewer device 6, moves them in the desired positions, with the aim for them to be invested by the laser radiation of the welding device 2 and to thus make the weld.

During the whole welding process, the operator can freely rotate his/her head or change his/her posture still being able to observe the objects 10 during the welding process. Therefore, if the operator continues to keep his/her hands inside the process chamber 3 through the openings 15, he/she can continue to carry out the welding in an ergonomic position.

During the process, the process parameters transmitted to the viewer device 6 by the electronic control unit 20 are shown to the operator on the lens elements 6b. Consequently, the operator can adjust such process parameters by means of the process parameters adjustment device 7.

Specifically, according to an embodiment not illustrated, the operator can adjust the process parameters by means of the vocal command device. More specifically, the operator utters a message relative to the adjustment of one or more process parameters. The message is thus detected by the microphone of the process parameters adjustment device 7 and transmitted to the control unit 20, which interprets the message and adjusts the one or the more process parameters selected depending on the information contained in the message.

The present invention also concerns a method of vision of manual welding operations on the welding workstation 1. Specifically, the vision method comprises the steps of i) capturing the images of the objects 10 to be welded through the image acquisition device 5 during the welding operations and ii) transmitting the images captured by the image acquisition device 5 to the viewer device 6, which is worn by the operator, with the aim to show the latter the captured images of the objects 10 to be welded during the welding operations.

More specifically, the transmission of the images which is performed in the step ii) is simultaneous with the capturing of the images which is performed in the step i). In this manner, the operator can view in real time the objects 10 subjected to the welding operations through the viewer device 6.

The present invention also concerns a method of updating an existing welding workstation for carrying out manual welding operations comprising:
- a welding device 2 adapted to provide the necessary heat and/or pressure for welding objects 10;
- a process chamber 3, within which the welding process is carried out by means of the welding device 2; and
- a microscope, which enables the operator to observe the inside of the process chamber 3 during the manual welding operations.

The method comprises the steps of i) installing in the workstation, i.e. mounting on a portion of the workstation, an image acquisition device 5, in addition to the microscope present in the workstation, configured to capture images of the objects 10 to be welded during the welding operations in the existing welding workstation; and ii) electrically connecting a viewer device 6 wearable by an operator carrying out the manual welding operations to the image acquisition device 5.

Specifically, the image acquisition device 5 comprises a video camera, an objective which is positioned at an eyepiece of said microscope of said workstation. More specifically, the objective of the video camera is positioned at the eyepiece, after removing the lens of the eyepiece of the microscope closest to said eyepiece.

Based on the foregoing, the advantages of the welding workstation 1, of the kit 100 and of the method according to the invention are apparent.

Since the vision system 4 comprises the image acquisition device 5 and the wearable viewer device 6, which shows the captured images of the objects 10 to be welded to the operator, the operator can freely change his/her posture while carrying out the welding. In fact, the operator is not obliged to remain in a fixed position with his/her eyes in contact with the eyepieces of a microscope or in visual contact with a non-wearable and fixed screen. On the contrary, the operator can, for example, freely rotate his/her head without losing the visual contact with the images captured by the image acquisition device 5 or sit with his/her back straight, without having to curve over the eyepieces of the microscope. Consequently, the ergonomic trim of the whole welding workstation 1 is significantly improved. It has been observed, in particular, that a greater comfort of the operator of the welding workstation 1 also corresponds to a greater level of productivity.

Moreover, since the images of the objects 10 during the welding operation are shown to the operator through the viewer device 6 and not through a non-wearable fixed screen, the risk of glare due to the light sources present in the surrounding environment is minimized.

It has been further observed that the visibility of the work area inside the process chamber 3 is generally improved with respect to the visibility obtainable through a non-wearable fixed screen. In fact, the operator obtains a three-dimensional perception of the objects 10 to be welded comparable to that obtainable with the eyepieces of a microscope.

The embodiment wherein the viewer device 6 is electrically connected to the image acquisition device 5 by means of an electro-magnetic connection is even more advantageous, since the operator is not constrained in his/her movements by the length of the wired connection.

It is possible to draw advantage also from the fact that the image acquisition device 5 can be electrically connected to a plurality of viewer devices 6 and/or of screens 16. In fact, in this manner, the carrying out of the process can be projected, for example, for educational purposes.

Moreover, since the viewer device 6 is wearable in addition to possible reading glasses worn by the operator, the operator is not obliged to take off his/her reading glasses before wearing the viewer device 6. Consequently, the vision system 4 is remarkably more ergonomic than the known vision systems. In fact, the operator that wears reading glasses will not be obliged to adjust the focusing settings of the viewer device 6 for adapting it to his/her sight.

Furthermore, the embodiment not illustrated wherein the process parameters adjustment device 7 comprises a vocal command device is particularly advantageous, since the operator can adjust the process parameters without having to interrupt the vision of the inside of the process chamber 3 and without having to use his/her hands.

Finally, it is clear that modifications and variants can be made to the welding workstation 1, to the method of vision, and to the method of updating embodied according to the present invention, without thereby departing from the scope of protection defined by the claims.

## Claims

1. Welding workstation (1) for carrying out manual welding operations comprising:
- a welding device (2) adapted to provide the necessary heat and/or pressure for welding objects (10);
**characterised in that**:
said welding device (2) comprising a laser source;
and **in that** the welding workstation (1) further comprises:
- a vision system (4) configured to show said objects (10) during the welding operations to an operator carrying out the welding operations;
said vision system (4) comprising, in turn:
- image acquisition means (5) configured to capture images of said objects (10) during the welding operations; and
- viewer means (6) wearable by the operator and electrically connected to said image acquisition means (5); said viewer means (6) being configured to show the images captured by said image acquisition means (5) to the operator;
wherein said image acquisition means (5) comprise an objective arranged coaxially to said laser source.

2. Welding workstation according to claim 1 further comprising a process chamber (3) within which the welding operations of said objects (10) are carried out by means of said welding device (2);
said vision system (4) being configured to show said objects (10) arranged in said process chamber (3) and/or to show said process chamber (3) during the welding operations to the operator.

3. Welding workstation according to claim 1 or 2, wherein said viewer means (6) comprise augmented reality glasses.

4. Welding workstation according to any one of the foregoing claims, wherein said image acquisition means (5) comprise a digital microscope or a video camera capable of capturing highly magnified images.

5. Welding workstation according to any one of the foregoing claims, wherein said viewer means (6) are electrically connected to said image acquisition means (5) by means of an electro-magnetic connection or a wired connection.

6. Welding workstation according to any one of the foregoing claims, wherein said image acquisition means (5) are electrically connected to a plurality of viewer means (6) .

7. Welding workstation according to any one of the foregoing claims further comprising an electronic control unit (20) electrically connected to said welding device (2) to control the process parameters of the welding operations and to said viewer means (6);
said viewer means (6) being configured to receive, in use, a signal relative to said process parameters from said electronic control unit (20) and to enable the operator to view and/or adjust said process parameters.

8. Welding workstation according to claim 7, comprising a device (7) for adjusting the process parameters which is electrically and operatively connected to said electronic control unit (20); said device (7) comprising a vocal command device.

9. Welding workstation according to any one of claims 3 to 8, wherein said augmented reality glasses comprise, in turn, a mount (6a) and two lens elements (6b) carried by said mount (6a);
each said lens element (6b) comprising a screen, on which, in use, the images captured by said image acquisition device (5) are reproduced;
said viewer means (6) comprising a covering removably couplable to said augmented reality glasses.

10. Method of updating a welding workstation for carrying out manual welding operations; said welding workstation comprising:
- a welding device (2) adapted to provide the necessary heat and/or pressure for welding objects (10);
- a process chamber (3), within which the welding process is carried out by means of said welding device (2); and
- a microscope, which enables the operator to observe, in use, the inside of said process chamber (3) during said manual welding operations;
**characterised in that** said welding device (2) comprises a laser source; 1
and, **in that** 1
said method comprises the steps of:
i) installing image acquisition means (5) configured to capture images of objects (10) to be welded during said welding operations in said welding workstation; and
ii) electrically connecting viewer means (6) wearable by an operator carrying out the manual welding operations to said image acquisition means (5); said viewer means (6) being configured to show the images captured by said image acquisition means (5) to the operator;
said method being **characterized in that** it comprises the further step iii) of arranging an objective of said image acquisition means (5) coaxially to said laser source.

11. Method according to claim 10, wherein said image acquisition means (5) comprise a video camera;
wherein said step i) comprises the step iv) of positioning said objective of said video camera at an eyepiece of said microscope of said workstation.

12. Method of vision of manual welding operations on a welding workstation (1),
the method being **characterised by** the following steps:
i) capturing images of objects (10) to be welded during the welding operations by image acquisition means (5); said welding operations being carried out by means of a welding device (2) of said welding workstation (1) adapted to provide the necessary heat and/or pressure for welding said objects (10) and comprising a laser source; said image acquisition means (5) comprising an objective; and
ii) transmitting the images captured during said step i) to viewer means (6) wearable by an operator carrying out the welding operations, so as to show such captured images to the operator;
wherein during said step i), said objective is arranged coaxially to said laser source.

## Patentansprüche

1. Schweißarbeitsplatz (1) zum Durchführen manueller Schweißvorgänge, umfassend:
- eine Schweißvorrichtung (2), die dazu geeignet ist, die notwendige Hitze und/oder den notwendigen Druck zum Schweißen von Objekten (10) bereitzustellen;
**dadurch gekennzeichnet, dass:**
die Schweißvorrichtung (2) eine Laserquelle umfasst; und
**dadurch, dass** der Schweißarbeitsplatz (1) ferner umfasst:
- ein Sichtprüfungssystem (4), das dazu konfiguriert ist, die Objekte (10) während der Schweißvorgänge einem Bediener anzuzeigen, der die Schweißvorgänge durchführt;
wobei das Sichtprüfungssystem (4) wiederum umfasst:
- Bilderfassungsmittel (5), die dazu konfiguriert sind, während der Schweißvorgänge Bilder der Objekte (10) aufzunehmen; und
- ein vom Bediener tragbares und mit dem Bilderfassungsmittel (5) elektrisch verbundenes Betrachtungsmittel (6); wobei das Betrachtungsmittel (6) dazu konfiguriert ist, dem Bediener die von der Bilderfassungseinrichtung (5) erfassten Bilder anzuzeigen;
wobei das Bilderfassungsmittel (5) ein Objektiv umfassen, das koaxial zur Laserquelle angeordnet ist.

2. Schweißarbeitsplatz nach Anspruch 1, ferner umfassend eine Prozesskammer (3), in der die Schweißvorgänge der Objekte (10) mittels der Schweißvorrichtung (2) durchgeführt werden;
wobei das Sichtprüfungssystem (4) dazu konfiguriert ist, die in der Prozesskammer (3) angeordneten Objekte (10) anzuzeigen und/oder dem Bediener die Prozesskammer (3) während der Schweißvorgänge zu zeigen.

3. Schweißarbeitsplatz nach Anspruch 1 oder 2, wobei das Betrachtungsmittel (6) eine Erweiterte-Realitätsbrille umfassen.

4. Schweißarbeitsplatz nach einem der vorstehenden Ansprüche, wobei das Bilderfassungsmittel (5) ein digitales Mikroskop oder eine Videokamera umfasst, die stark vergrößerte Bilder aufnehmen kann.

5. Schweißarbeitsplatz nach einem der vorstehenden Ansprüche, wobei das Betrachtungsmittel (6) über eine elektromagnetische Verbindung oder eine Kabelverbindung elektrisch mit den Bilderfassungsmitteln (5) verbunden sind.

6. Schweißarbeitsplatz nach einem der vorstehenden Ansprüche, wobei das Bilderfassungsmittel (5) elektrisch mit einer Vielzahl von Betrachtungsmitteln (6) verbunden ist.

7. Schweißarbeitsplatz nach einem der vorstehenden Ansprüche, ferner umfassend eine elektronische Steuereinheit (20), die elektrisch verbunden ist mit der Schweißvorrichtung (2), um die Prozessparameter der Schweißvorgänge zu steuern, und mit dem Betrachtungsmittel (6);
wobei das Betrachtungsmittel (6) so konfiguriert ist, dass es im Betrieb ein Signal bezüglich der Prozessparameter von der elektronischen Steuereinheit (20) empfängt und es dem Bediener ermöglicht, die Prozessparameter anzuzeigen und/oder anzupassen.

8. Schweißarbeitsplatz nach Anspruch 7, umfassend eine Vorrichtung (7) zum Einstellen der Prozessparameter, die elektrisch und betriebsmäßig mit der elektronischen Steuereinheit (20) verbunden ist; wobei die Vorrichtung (7) eine Vorrichtung zur Sprachsteuerung umfasst.

9. Schweißarbeitsplatz nach einem der Ansprüche 3 bis 8, wobei die Erweiterte-Realitätsbrille wiederum eine Halterung (6a) und zwei von der Halterung (6a) getragene Linsenelemente (6b) umfasst;
wobei jedes Linsenelement (6b) einen Bildschirm umfasst, auf dem im Gebrauch die von der Bilderfassungsvorrichtung (5) erfassten Bilder wiedergegeben werden;
wobei das Betrachtungsmittel (6) eine Abdeckung umfasst, die abnehmbar mit der Erweiterten-Realitätsbrille verbunden werden kann.

10. Verfahren zum Aufrüsten eines Schweißarbeitsplatzes zum Durchführen manueller Schweißvorgänge; wobei der Schweißarbeitsplatz umfasst:
- eine Schweißvorrichtung (2), die dazu geeignet ist, die notwendige Hitze und/oder den notwendigen Druck zum Schweißen von Objekten (10) bereitzustellen;
- eine Prozesskammer (3), innerhalb der der Schweißprozess mittels der Schweißvorrichtung (2) durchgeführt wird; und
- ein Mikroskop, das es dem Bediener ermöglicht, während der manuellen Schweißvorgänge das Innere der Prozesskammer (3) zu beobachten;
**dadurch gekennzeichnet, dass** die Schweißvorrichtung (2) eine Laserquelle umfasst;
und **dadurch, dass** das Verfahren die folgenden Schritte umfasst:
i) Installieren von Bilderfassungsmitteln (5), die dazu konfiguriert sind, während der Schweißvorgänge in dem Schweißarbeitsplatz Bilder von zu schweißenden Objekten (10) aufzunehmen; und
ii) elektrisches Verbinden eines von einem die manuellen Schweißvorgänge durchführenden Bediener tragbaren Betrachtungsmittels (6) mit dem Bilderfassungsmittel (5); wobei das Betrachtungsmittel (6) dazu konfiguriert ist, dem Bediener die von der Bilderfassungseinrichtung (5) erfassten Bilder anzuzeigen;
wobei die Methode **dadurch gekennzeichnet, dass** es den weiteren Schritt iii) des Anordnens eines Objektivs der Bilderfassungseinrichtung (5) koaxial zur Laserquelle umfasst.

11. Verfahren nach Anspruch 10, wobei die Bilderfassungsmittel (5) eine Videokamera umfassen;
wobei der Schritt i) den Schritt iv) des Positionierens des Objektivs der Videokamera an einem Okular des Mikroskops der Arbeitsstation umfasst.

12. Verfahren zur Sichtprüfung von manuellen Schweißvorgängen an einem Schweißarbeitsplatz (1), wobei das Verfahren **gekennzeichnet ist durch** die folgenden Schritte:
i) Aufnehmen von Bildern der zu schweißenden Objekte (10) während der Schweißvorgänge durch Bilderfassungsmittel (5); wobei die Schweißvorgänge mittels einer Schweißvorrichtung (2) des Schweißarbeitsplatzes (1) durchgeführt werden, die dazu geeignet ist, die notwendige Hitze und/oder den notwendigen Druck zum Schweißen der Objekte (10) bereitzustellen, und die eine Laserquelle umfasst; wobei die Bilderfassungseinrichtung (5) ein Objektiv umfasst; und
ii) Übertragen der während des Schritts i) erfassten Bilder an ein Betrachtungsmittel (6), das von einem Bediener getragen werden kann, der die Schweißvorgänge durchführt, um dem Bediener die erfassten Bilder anzuzeigen;
wobei während des Schritts i) das Objektiv koaxial zur Laserquelle angeordnet ist.

## Revendications

1. Poste de travail de soudage (1) permettant de réaliser des opérations manuelles de soudage comprenant :
- un dispositif de soudage (2) adapté pour fournir la chaleur et/ou la pression nécessaires pour souder des objets (10) ;
**caractérisé en ce que** :
ledit dispositif de soudage (2) comprend une source laser ; et **en ce que** le poste de travail de soudage (1) comprend en outre :
- un système de vision (4) configuré pour présenter lesdits objets (10) pendant les opérations de soudage à un opérateur réalisant les opérations de soudage ;
ledit système de vision (4) comprenant, à son tour :
- un moyen d'acquisition d'images (5) configuré pour capturer des images desdits objets (10) pendant les opérations de soudage ; et
- un moyen de visualisation (6) pouvant être porté par l'opérateur et connecté électriquement audit moyen d'acquisition d'images (5) ; ledit moyen de visualisation (6) étant configuré pour présenter les images capturées par ledit moyen d'acquisition d'images (5) à l'opérateur ;
dans lequel ledit moyen d'acquisition d'images (5) comprend un objectif disposé coaxialement à ladite source laser.

2. Poste de travail de soudage selon la revendication 1, comprenant en outre une chambre de traitement (3) à l'intérieur de laquelle les opérations de soudage desdits objets (10) sont réalisées au moyen dudit dispositif de soudage (2) ;
ledit système de vision (4) étant configuré pour présenter lesdits objets (10) disposés dans ladite chambre de traitement (3) et/ou pour présenter ladite chambre de traitement (3) pendant les opérations de soudage à l'opérateur.

3. Poste de travail de soudage selon la revendication 1 ou 2, dans lequel ledit moyen de visualisation (6) comprend des lunettes de réalité augmentée.

4. Poste de travail de soudage selon l'une quelconque des revendications précédentes, dans lequel ledit moyen d'acquisition d'images (5) comprend un microscope numérique ou une caméra vidéo capable de capturer des images fortement agrandies.

5. Poste de travail de soudage selon l'une quelconque des revendications précédentes, dans lequel ledit moyen de visualisation (6) est connecté électriquement audit moyen d'acquisition d'images (5) au moyen d'une connexion électromagnétique ou d'une connexion câblée.

6. Poste de travail de soudage selon l'une quelconque des revendications précédentes, dans lequel ledit moyen d'acquisition d'images (5) est connecté électriquement à une pluralité de moyens de visualisation (6).

7. Poste de travail de soudage selon l'une quelconque des revendications précédentes, comprenant en outre une unité de commande électronique (20) connectée électriquement audit dispositif de soudage (2) pour commander les paramètres de processus des opérations de soudage et audit moyen de visualisation (6) ;
ledit moyen de visualisation (6) étant configuré pour recevoir, en cours d'utilisation, un signal relatif auxdits paramètres de traitement de la part de ladite unité de commande électronique (20) et pour permettre à l'opérateur de visualiser et/ou d'ajuster lesdits paramètres de traitement.

8. Poste de travail de soudage selon la revendication 7, comprenant un dispositif (7) permettant d'ajuster les paramètres de traitement qui est connecté électriquement et de manière opérationnelle à ladite unité de commande électronique (20) ; ledit dispositif (7) comprenant un dispositif de commande vocale.

9. Poste de travail de soudage selon l'une quelconque des revendications 3 à 8, dans lequel lesdites lunettes de réalité augmentée comprennent, à leur tour, une monture (6a) et deux éléments de lentille (6b) portés par ladite monture (6a) ;
chaque dit élément de lentille (6b) comprenant un écran sur lequel sont reproduites, en cours d'utilisation, les images capturées par ledit dispositif d'acquisition d'images (5) ;
ledit moyen de visualisation (6) comprenant une couverture pouvant être accouplée de manière amovible auxdites lunettes de réalité augmentée.

10. Procédé de mise à jour d'un poste de travail de soudage permettant de réaliser des opérations manuelles de soudage ; ledit poste de travail de soudage comprenant :
- un dispositif de soudage (2) adapté pour fournir la chaleur et/ou la pression nécessaires pour souder des objets (10) ;
- une chambre de traitement (3), à l'intérieur de laquelle le processus de soudage est réalisé au moyen dudit dispositif de soudage (2) ; et
- un microscope, qui permet à l'opérateur d'observer, en cours d'utilisation, l'intérieur de ladite chambre de traitement (3) pendant lesdites opérations manuelles de soudage ;
**caractérisé en ce que** ledit dispositif de soudage (2) comprend une source laser ;
et, **en ce que** ledit procédé comprend les étapes consistant à :
i) installer un moyen d'acquisition d'images (5) configuré pour capturer des images d'objets (10) à souder pendant lesdites opérations de soudage dans ledit poste de travail de soudage ; et
ii) connecter électriquement un moyen de visualisation (6) pouvant être porté par un opérateur réalisant les opérations manuelles de soudage audit moyen d'acquisition d'images (5) ; ledit moyen de visualisation (6) étant configuré pour présenter les images capturées par ledit moyen d'acquisition d'images (5) à l'opérateur ;
ledit procédé étant **caractérisé en ce qu'**il comprend l'étape supplémentaire iii) consistant à disposer un objectif dudit moyen d'acquisition d'images (5) coaxialement à ladite source laser.

11. Procédé selon la revendication 10, dans lequel ledit moyen d'acquisition d'images (5) comprend une caméra vidéo ;
dans lequel ladite étape i) comprend l'étape iv) consistant à positionner ledit objectif de ladite caméra vidéo au niveau d'un oculaire dudit microscope de ladite station de travail.

12. Procédé de vision d'opérations manuelles de soudage sur un poste de travail de soudage (1), le procédé étant **caractérisé par** les étapes suivantes consistant à :
i) capturer des images d'objets (10) à souder pendant les opérations de soudage par un moyen d'acquisition d'images (5) ; lesdites opérations de soudage étant réalisées au moyen d'un dispositif de soudage (2) dudit poste de travail de soudage (1) adapté pour fournir la chaleur et/ou la pression nécessaires au soudage desdits objets (10) et comprenant une source laser ; ledit moyen d'acquisition d'images (5) comprenant un objectif ; et
ii) transmettre les images capturées au cours de ladite étape i) à un moyen de visualisation (6) pouvant être porté par un opérateur réalisant les opérations de soudage, afin de présenter les telles images capturées à l'opérateur ;
dans lequel, au cours de ladite étape i), ledit objectif est disposé coaxialement à ladite source laser.
